# EUROPEAN PATENT SPECIFICATION

(11) **EP 3 551 245 B1**
(45) Date of publication and mention of the grant of the patent: **06.01.2021**
(21) Application number: 17826441.2
(22) Date of filing: 05.12.2017
(51) Int. Cl.: A61M 1/00, A61M 25/00

(54) **THORACIC DRAINAGE SYSTEM**
THORAXDRAINAGESYSTEM
SYSTÈME DE DRAINAGE THORACIQUE

(30) Priority: 12.12.2016 IT 201600124793
(43) Date of publication of application: 16.10.2019
(73) Proprietor: Redax S.p.A., 46025 Poggio Rusco (MN) (IT)
(72) Inventor: GIBERTONI, Lucio, 41037 Mirandola (IT); GIBERTONI, Andrea, 41039 San Possidonio (IT)
(74) Representative: Modiano, Micaela Nadia
(86) International application number: PCT/EP2017/081500
(87) International publication number: WO 2018/108625

(56) References cited:
- US-A- 5 360 414
- US-A1- 2004 006 331
- US-A1- 2014 163 532
- US-A1- 2015 231 361
- US-B1- 6 299 593

## Description

The present invention relates to a thoracic drainage system for the drainage of air and/or liquids formed as a consequence of surgical procedures or traumas, from the thoracic cavity of a patient, in order to avoid their accumulation Examples are described in US 2015/0231361 A1 or US 6,299,593 B1.

In general, thoracic drainage systems of the known type comprise a thoracic drainage catheter, i.e., a flexible tube that is provided by using transparent and sterile plastic materials, such as for example PVC (polyvinyl chloride) or silicone, and is inserted, through an incision, in the thorax of the patient until it reaches the pleural space in the thoracic cavity.

This drainage catheter constitutes the distal element of a thoracic drainage system and is therefore used to reach and remove the fluids, i.e., air and/or liquids, that have accumulated in the thoracic cavity.

In particular, usually air tends to accumulate in the apical region of the thoracic cavity of the patient, while liquids, such as for example blood, tend to accumulate in the basal region of the thoracic cavity of the patient.

Various types of drainage catheter for thoracic drainage systems are currently known. One of these thoracic drainage catheters of the known type is provided with a plurality of holes in its terminal portion, which is arranged in the apical region of the thoracic cavity of the patient, thus allowing the immediate outflow of the air toward the collection chamber, the liquids instead remaining deposited in the basal region of the thoracic cavity.

By using this known thoracic drainage catheter, the evacuation of the liquids, initially deposited in the basal region of the thoracic cavity, thus occurs when the lung, by expanding by virtue of the outflow of the air, pushes said liquids toward the apical region of said thoracic cavity, i.e., toward the holes that are present in the end portion of the drainage catheter and therefore toward the outlet of the catheter, up to the collection chamber.

It is evident that by using this thoracic drainage catheter of the known type, provided with holes in its terminal portion, the air and the liquids that are present in the thoracic cavity of the patient are evacuated alternately and not simultaneously, since the air exits first and the liquids exit only at a later time.

A different thoracic drainage catheter of the known type has, as a replacement of the holes in the terminal portion, one or more grooves which are longitudinal with respect to the thoracic drainage catheter. This type of drainage catheter has been conceived in an attempt to allow the simultaneous evacuation of air and liquids from the thoracic cavity of the patient by using a single drainage catheter, but in this case the outflow of the liquids from the basal region of the thoracic cavity occurs immediately and the outflow of the air from the apical region of said thoracic cavity occurs only subsequently.

It is therefore evident that even by using this grooved thoracic drainage catheter of a known type the simultaneous outflow of the air and of the liquids that are present in the thoracic cavity of the patient does not occur, since the liquids exit first and the air exits only at a later time.

In view of the above, these currently known thoracic drainage catheters are not free from drawbacks, which include the fact that they do not allow the simultaneous and continuous evacuation of the air and of the liquids that are present in the thoracic cavity of the patient.

Another drawback of these thoracic drainage catheters of the known type resides in that since they do not allow continuous evacuation of the air in the collection chamber, in practice they prevent medical staff from viewing immediately any air leaks of the patient.

One solution to the drawback described above, related to the evacuation from the thoracic cavity of the patient of air and liquids in two distinct and alternating steps, provides for positioning a pair of drainage catheters, both provided with a plurality of holes in their terminal portion, inside the thoracic cavity, one arranged in the apical region of the pleural space and the other one in the basal region.

The first drainage catheter, arranged in the apical region, allows the evacuation of the air, while the second drainage catheter, arranged in the basal region, allows the evacuation of the liquids. Both drainage catheters then merge in a three-way or Y-shaped connector and the fluids that they carry continue toward the collection chamber in a single tube.

Therefore, by using a pair of known thoracic drainage catheters the air and liquids that are present in the thoracic cavity of the patient can be evacuated simultaneously.

The drawback of these thoracic drainage catheters of the known type resides in that the only way to ensure simultaneous and continuous evacuation of the air and of the liquids that are present in the thoracic cavity of the patient is to use a pair of known drainage catheters, but this solution leads to a significant increase in the intensity of the pain felt by the patient, as well as to additional difficulties in the already complex management of the thoracic drainage system.

A further solution to the drawbacks described above provides for the use of a so-called coaxial drainage catheter, i.e., comprising two coaxial ports, wherein the internal port is assigned to the drainage of air while the external port is assigned to the drainage of liquids.

In particular, the catheter has, in the distal portion arranged to reside in the apical region of the pleural space, where air is present, a plurality of openings or windows which connect the internal port to the external space. In the proximal portion, arranged to reside in the basal region of the pleural space, where liquids are instead most present, the external space is connected only to the external port, by means of other openings or windows. In this manner the drainage catheter is capable of draining simultaneously and continuously both air and liquids through the two defined preferential paths.

However, even this last solution is not free from drawbacks.

In particular, the provision of drainage catheters provided with appropriate windows is rather complicated, time-consuming and expensive, since it requires a succession of different processes.

Furthermore, another drawback of drainage systems that provide for the use of the above-cited coaxial drainage catheters resides in that the air and the liquids are made to flow out together toward the collection chamber, which is often at a certain distance from the patient, through a drain tube.

If the drain tube becomes occluded, for example due to prolonged use of the thoracic drainage system, or due to the accumulation of liquids in a bend that has formed along the drain tube, air evacuation on the part of the patient is prevented. Indeed, if the patient who has air leaks to be evacuated is unable to exert such a positive pressure as to release the occlusion of the drain tube, severe respiratory and/or cardiac failures can arise, leading even to patient death.

One solution to the drawback cited above resides in applying to the drainage system, downstream of the drainage catheter, a separation filter, so as to separate the liquid phase from the gaseous phase and keep the two paths separate up to the collection chamber. In this manner the portion of the drain tube downstream of the separation filter has a preferential path for the air.

The fact of providing a separation filter, however, entails a considerable increase in the complexity and cost of said drainage system, since it is necessary to provide two drain tubes, one for the liquids and the other one for the air, as well as two connectors for connection to the collection chamber, which must indeed have a double coupling, while the drainage systems currently used commonly have a single coupling.

The aim of the present invention is to overcome the limitations of the background art described above, devising a thoracic drainage system that allows to obtain better effects than those obtainable with thoracic drainage systems of the known type, allowing the simultaneous and continuous evacuation of the fluids, i.e., of the air and of the liquids, that are present in the pleural space of the patient.

Within this aim, an object of the present invention is to conceive a thoracic drainage system that facilitates a faster expansion of the lung by virtue of the simultaneous evacuation of the air and of the liquids, with the consequent reduction of the hospitalization times of the patient.

A further object of the present invention is to conceive a thoracic drainage system that minimizes the pain felt by the patient as well as the possibility of external contamination of said patient.

Another object of the present invention is to devise a thoracic drainage system that allows continuous evacuation of the air in the collection chamber, allowing medical staff to visualize immediately any air leaks of the patient and providing medical staff with additional information on the air leaks of the patient.

Another object of the present invention is to conceive a thoracic drainage system that allows to reduce the workload of the medical and nursing staff.

Another object of the present invention is to provide a thoracic drainage system that minimizes and possibly eliminates the unwanted occlusion events of the air and liquid drain tube.

Another object of the invention is to devise a thoracic drainage system that substantially has standard components.

A further object of the invention is to devise a thoracic drainage system the main components of which, such as for example the drainage catheter and the drain tube, can be provided by means of production methods that can be fully industrialized, in large numbers, at reduced costs, and in short times.

A still further object of the present invention is to provide a thoracic drainage system that is highly reliable, relatively simple to provide and at modest costs.

This aim, as well as these and other objects which will become better apparent hereinafter, are achieved by a thoracic drainage system, comprising a drainage catheter adapted to be inserted in the pleural space of a patient in order to drain air and/or liquids that are present in said pleural space, and a drain tube, which is connected to said drainage catheter by means of a connector, which is adapted to make said air and/or said drained liquids flow out via said drainage catheter to a collection chamber, characterized in that said drainage catheter comprises, at its distal end, a plurality of longitudinally extended channels which are open radially toward the outside of said drainage catheter and are adapted to drain said liquids that are present in said pleural space, and, at its proximal end, at least one port for the drainage of said liquids, in which said open channels merge, said drainage catheter comprising a port for the drainage of said air that is present in said pleural space, said port for the drainage of said air comprising, at said distal end of said drainage catheter, a plurality of radial holes adapted to be crossed by said air that is present in said pleural space, said drain tube comprising, substantially along its entire longitudinal extension, a first outflow port which is connected, by means of said connector, to said at least one port for the drainage of said liquids and a second outflow port which is connected, by means of said connector, to said port for the drainage of said air.

Further characteristics and advantages of the invention will become better apparent from the description of a preferred but not exclusive embodiment of a thoracic drainage system according to the invention, illustrated by way of nonlimiting example in the accompanying drawings, wherein:
Figure 1 is a perspective view of a first embodiment of a thoracic drainage system according to the present invention;
Figure 2 is a sectional view of the thoracic drainage system of Figure 1, according to the present invention;
Figure 3 is a perspective view of the distal end of a drainage catheter that belongs to the thoracic drainage system of Figure 1, according to the present invention;
Figure 4 is a different perspective view of the distal end of the drainage catheter that belongs to the thoracic drainage system of Figure 1, according to the present invention;
Figure 5 is a further sectional perspective view of the distal end of the drainage catheter that belongs to the thoracic drainage system of Figure 1, according to the present invention;
Figure 6 is a perspective view of the proximal end of the drainage catheter that belongs to the thoracic drainage system of Figure 1, according to the present invention;
Figure 7 is a sectional exploded view of a connector that belongs to the thoracic drainage system of Figure 1, according to the present invention;
Figure 8 is a perspective view of the distal end of a drain tube which belongs to the thoracic drainage system of Figure 1, according to the present invention;
Figure 9 is a perspective view of a second embodiment of a thoracic drainage system according to the present invention;
Figure 10 is a sectional view of the thoracic drainage system of Figure 9, according to the present invention;
Figure 11 is a perspective view of a drainage catheter that belongs to the thoracic drainage system of Figure 9, according to the present invention;
Figure 12 is a sectional exploded view of a connector that belongs to the thoracic drainage system of Figure 9, according to the present invention;
Figure 13 is a side view of a drainage catheter and of a drain tube that belong to a third embodiment of a thoracic drainage system, according to the present invention;
Figure 14 is a sectional view of the drainage catheter shown in Figure 13, taken along the plane XIV;
Figure 15 is a sectional view of the drainage catheter shown in Figure 13, taken along the plane XV;
Figure 16 is a sectional view of the drain tube shown in Figure 13, taken along the plane XVI.

With reference to the cited figures, the thoracic drainage system, generally designated by the reference numeral 1, comprises a drainage catheter 10 adapted to be inserted in the pleural space of a patient in order to drain air and/or liquids that are present in the pleural space, and a outflow tube 50, which is connected to the drainage catheter 10 by means of a connector 30 which is adapted to make the drained air and/or liquids flow out via the drainage catheter 10 to a collection chamber 60.

According to the invention, the drainage catheter 10 comprises, at its distal end 11, a plurality of longitudinally extended channels 13, 14 which are open radially toward the outside of the drainage catheter 10 and are adapted to drain the liquids that are present in the pleural space, and, at its proximal end 12, at least one port 15, 150 for the drainage of said liquids, in which the open channels 13, 14 merge. The drainage catheter 10 furthermore comprises a port 16 for the drainage of the air that is present in the pleural space. The air drainage port 16 comprises, at the distal end 11 of the drainage catheter 10, a plurality of radial holes 17 adapted to be crossed by the air that is present in the pleural space.

Furthermore, according to the invention, the outflow tube 50 comprises, substantially along its entire longitudinal extension, a first outflow port 51, which is connected, by means of the connector 30, to the liquid drainage port 15, 150, and a second outflow port 52, which is connected, again by means of the connector 30, to the air drainage port 16.

In this manner, the air and the liquids that are present in the pleural space can be drained in separate channels along the extension of both the drainage catheter 10 and the outflow tube 50.

The term "proximal" is understood to reference that which is proximate to the base of the drainage system 1, i.e., essentially, to the collection chamber 60, while the term "distal" is understood to reference that which is furthest from the base of the drainage system 1, and therefore closest to the patient.

Advantageously, both the drainage catheter 10 and the outflow tube 50 can be obtained by means of processes for the extrusion of materials such as plastic, by virtue of the particular geometry of their transverse cross-sections.

Advantageously, the radial holes 17 for the passage of air inside the air drainage port 16 are arranged in the most distal portion of the drainage catheter 10, which is designed to reach, during use, the apical region of the pleural space, where the air to be drained accumulates more easily. In this manner it is possible to ensure a continuous drainage of the air that is present in the pleural space toward the collection chamber 60.

The open channels 13, 14 advantageously are extended along a greater length than the drainage catheter 10, affecting also portions of the catheter 10 located downstream, i.e., more proximally, with respect to where the radial holes 17 are arranged. In particular, the open channels 13, 14 are extended in the portion of the drainage catheter 10 that is designed to reach, during use, the basal region of the pleural space where the liquids to be drained instead tend to accumulate by gravity.

These open channels 13, 14 therefore allow the entry of the liquids that are present in the basal region of the pleural space and their drainage toward the liquid drainage port 15, 150 into which said open channels 13, 14 are conveyed.

In this manner it is possible to ensure continuous drainage also of the liquids that are present in the pleural space, toward the collection chamber 60.

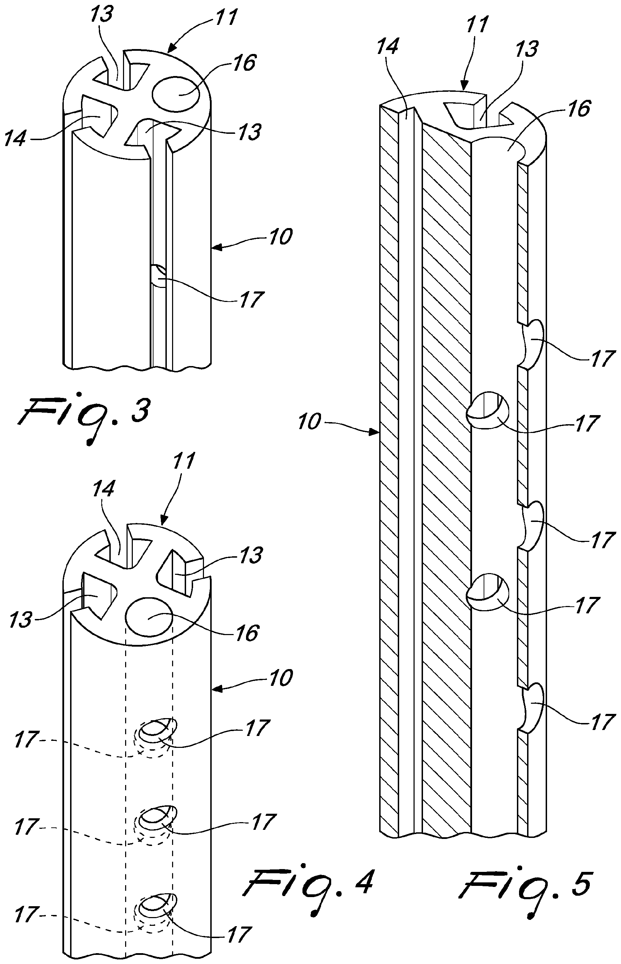

Advantageously, as shown in particular in Figures 3, 4 and 5, some radial holes 17 are directed radially toward the outside of the drainage catheter 10, while other radial holes 17 are directed toward at least one of the open channels 13, 14. For example, the air drainage port 16 can have radial holes 17 that are open toward the open channels 13 arranged laterally to said port 16.

In this manner, should the holes 17 that are directed toward the outside of the drainage catheter 10 become blocked, for example if they rest against the walls of the pleural space, air drainage would in any case be allowed through the holes that are directed toward the inside of the open channels 13.

Naturally, the number and/or breadth and/or arrangement of said radial holes 17 can vary in the various embodiments of the invention, while remaining within a value that does not affect negatively the very structure of the drainage catheter 10.

Advantageously, the drainage catheter 10 has, at the distal end 11, a substantially circular cross-section divided into at least two circular sectors. A first circular sector accommodates the air drainage port 16, while a second circular sector accommodates the open channels 13, 14.

In particular, as shown in Figures 3 and 4, the circular cross-section of the drainage catheter 10 can be divided into four circular sectors, of which one is occupied by the air drainage port 16 and the other three are occupied by the open channels 13, 14.

Advantageously, the drainage catheter 10 has, at the proximal end 12, a substantially circular cross-section divided into two circular sectors. One first circular sector accommodates the air drainage port 16, while a second circular sector accommodates the liquid drainage port 15, in which the open channels 13, 14 merge.

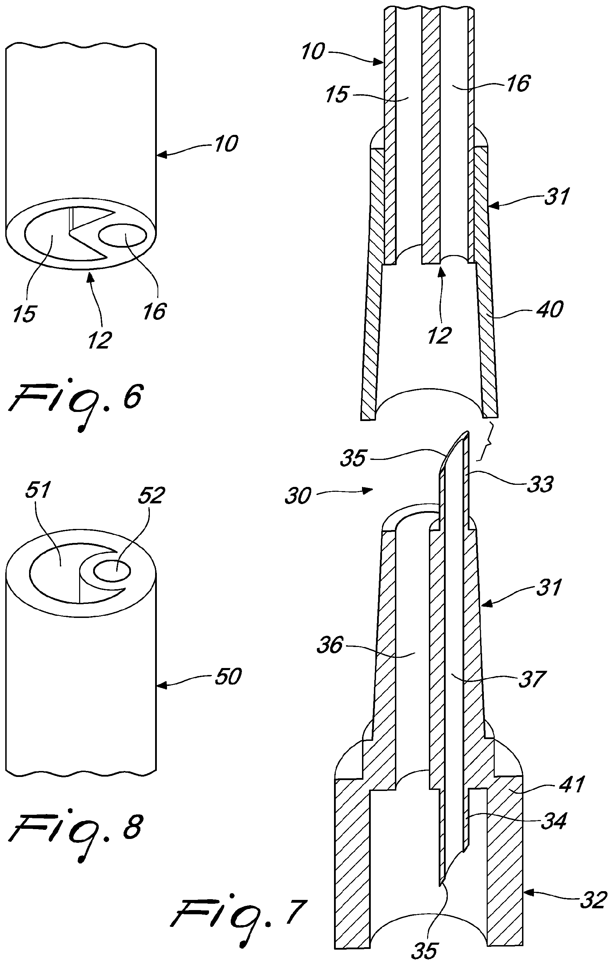

As shown in Figure 6, at the proximal end 12 the drainage catheter 10 has a circular cross-section divided into two sectors, wherein the first sector, which accommodates the air drainage port 16, occupies approximately one quarter of the area of the cross-sectional circle, while the liquid drainage port 15 occupies the remaining part, i.e., approximately three quarters, of the area of the cross-sectional circle.

In an axial portion between the distal end 11 and the proximal end 12 of the drainage catheter 10, the open channels 13, 14 become closed channels, i.e., not open outward, and merge in the liquid drainage port 15.

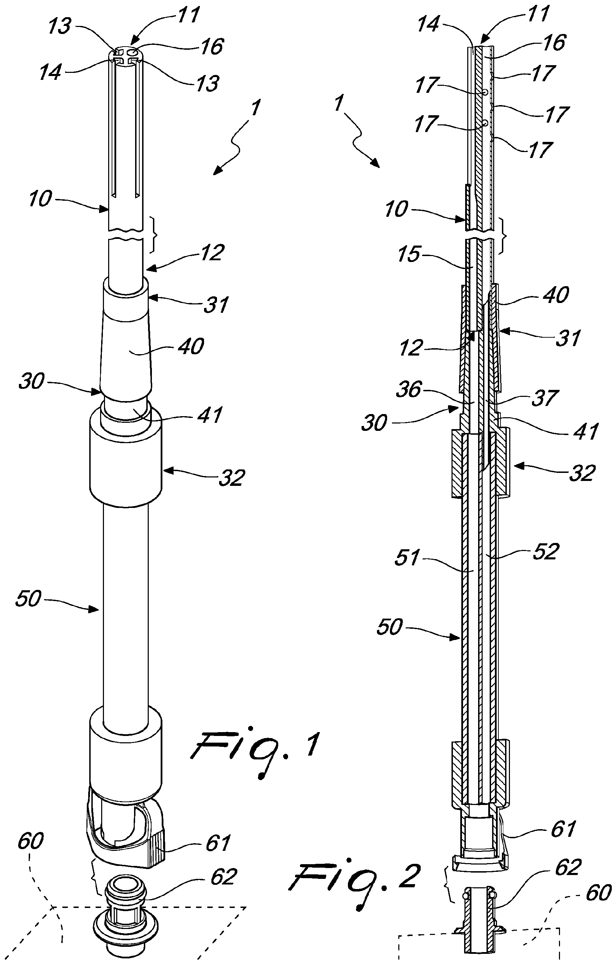

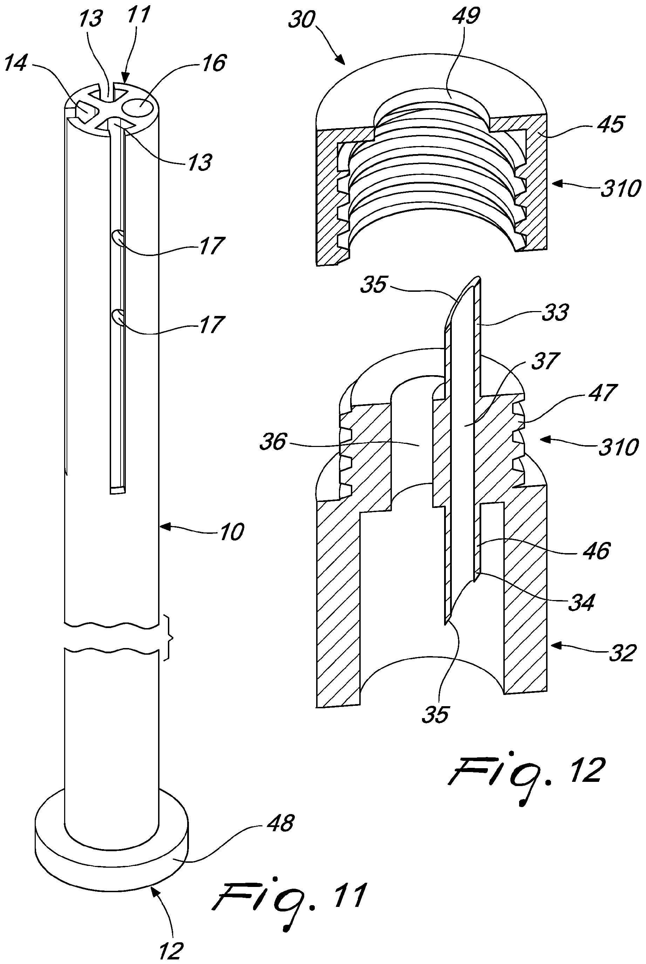

As shown in Figures 1 to 12, which relate to the first and second embodiments of the drainage system 1, the air drainage port 16 is in an off-centered position with respect to the longitudinal axis of the drainage catheter 10.

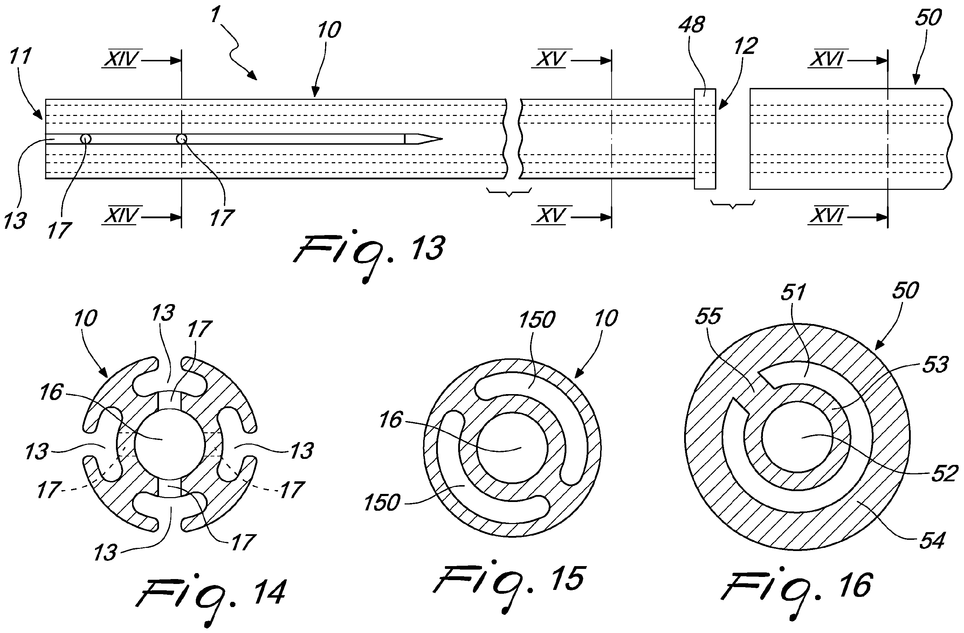

Advantageously, as shown in Figures 13 to 16, in a third embodiment of the drainage system 1 the drainage catheter 10 can have, at the distal end 11, a substantially circular cross-section, and the air drainage port 16 is advantageously arranged centrally with respect to said substantially circular cross-section, while the open channels 13, 14 are provided so as to be radially external with respect to the air drainage port 16.

For example, as shown in Figure 14, the drainage catheter 10 has, radially more externally, four open channels 13, while the air drainage port 16 is arranged at the center.

At the proximal end 12 it is possible to provide a pair of liquid drainage ports 150 which have, as shown in Figure 15, a substantially semiannular cross-section and are arranged radially external with respect to the air drainage port 16.

In this third embodiment of the drainage system 1, in the drainage catheter 10, between the distal end 11 and the proximal end 12, the open channels 13 become closed and merge in pairs in the two liquid drainage ports 150.

Advantageously, furthermore, in this third embodiment of the drainage system 1 the outflow tube 50 comprises an internal tube 53, which defines the second outflow port 52, and an external tube 54. Between the external tube 54 and the internal tube 53 there is an interspace that constitutes the first outflow port 51. Advantageously, the internal tube 53 is arranged substantially at the center of the external tube 54 and is associated therewith by means of a rib 55 extended longitudinally along substantially all of the outflow tube 50.

Advantageously, the connector 30 is provided with a first portion 31, 310, which can be associated with the drainage catheter 10 by interference or screwing, and a second portion 32, which is arranged opposite the first portion 31 and can be associated with the outflow tube 50 by interference and/or adhesive bonding.

Furthermore, the connector 30 advantageously comprises a first connecting duct 36 which is arranged to connect the liquid drainage port 15 to the first outflow port 51, and a second connecting duct 37 that is arranged to connect the air drainage port 16 to the second outflow port 52. Furthermore, the second connecting duct 37 comprises, at both of its ends, two cylindrical walls 33, 34 which protrude axially and are arranged to engage respectively in the air drainage port 16 and in the second outflow port 52.

Advantageously, the cylindrical walls 33, 34 comprise a guiding bevel 35 for engagement within the air drainage port 16 and within the second outflow port 52.

In a first embodiment of the drainage system 1, shown in Figures 1 to 8, the connector 30 comprises a sleeve 40 and a connecting body 41. The sleeve 40 is configured to retain together, by interference, the proximal end 12 of the drainage catheter 10 with the connecting body 41. The connecting body 41 is instead associated, by interference and/or adhesive bonding, with the outflow tube 50.

Preferably, the connector 30 is joined to the outflow tube 50 by means of chemical bonding methods.

The cylindrical walls 33, 34 for coupling in the air drainage port 16 and in the second outflow port 52 are advantageously provided by the connecting body 41.

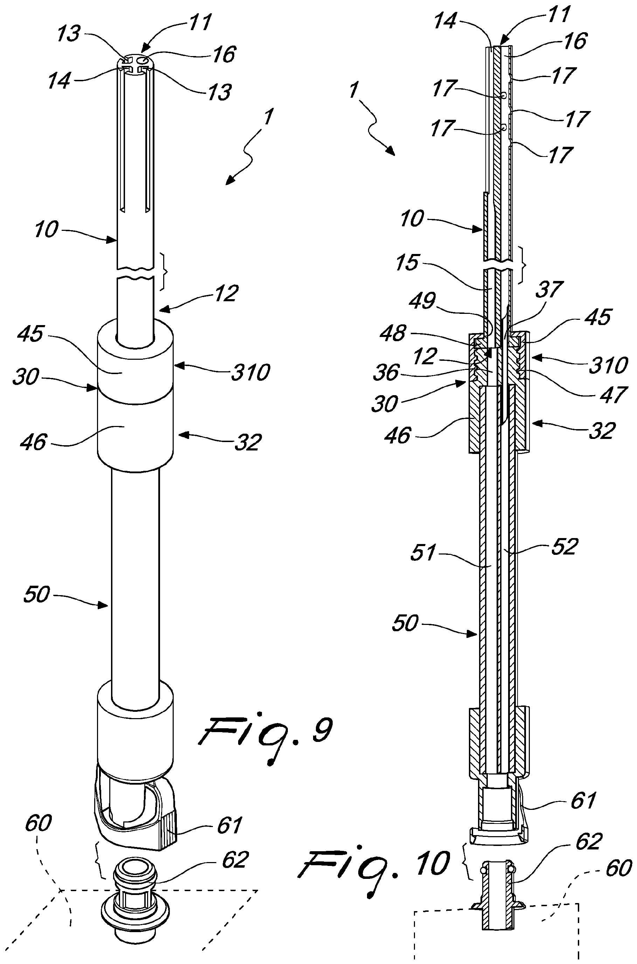

In a second embodiment of the drainage system 1, shown in Figures 9 to 12, the connector 30 comprises a threaded ring 45 and a connecting body 46, which comprises a threaded head 47.

The connecting body 46 is advantageously associated by interference with the outflow tube 50.

The drainage catheter 10 comprises, at its proximal end 12, a collar 48 which protrudes radially and is configured to be retained between the threaded ring 45 and the connecting body 46.

In particular, the threaded ring 45 is provided with a through hole 49 which has such a diameter that it can be crossed by the body of the drainage catheter 10 but not by the corresponding terminal collar 48.

The fastening of the threaded ring 45 on the threaded head 47 of the connecting body 46 allows to block the collar 48 and therefore the drainage catheter 10.

A quick coupling connector 61 is advantageously joined, for example by chemical bonding, in the proximal part of the outflow tube 50 and is configured to engage a corresponding connector 62 that is present on the collection chamber 60.

The quick coupling connector 61 is advantageously a connector of the standard type, which can be associated, at the operator's discretion, with collection systems of various kinds, such as for example mechanical collection systems, water-based systems, gravity systems, with centralized suction, with suction by means of a vacuum unit, or other collection systems provided with systems for recording intrapleural pressure data.

In practice it has been found that the invention achieves fully the intended aim and objects. In particular, it has been shown that the thoracic drainage system thus conceived allows to overcome the quality limitations of the background art, since it allows the simultaneous and continuous evacuation of the fluids, i.e., of the air and of the liquids, that are present in the thoracic cavity of the patient.

Another advantage of the thoracic drainage system according to the invention resides in that its main components, i.e., the drainage catheter, the drain tube and the corresponding connector, can be provided by means of methods that can be easily industrialized, such as for example extrusion and/or molding, without requiring complicated subsequent processes. In particular, the provision of the extruded components, i.e., of the drainage catheter and of the drain tube, does not require particular adhesive bonding or welding steps.

A further advantage of the thoracic drainage system according to the invention resides in that it minimizes and even eliminates the harmful occlusion events of the air and/or liquid evacuation channels, always ensuring a free path for the drainage of the air from the pleural space to the collection chamber.

Another advantage of the thoracic drainage system according to the invention resides in that it can be interfaced with collection chambers of the known and standard type and does not require the adoption of particular separation filters.

In particular, the thoracic drainage system according to the invention allows to record the data of the intrapleural pressures without variations that might be caused by the application of intermediate separation filters.

Another advantage of the thoracic drainage system according to the invention resides in that it facilitates a more rapid expansion of the lung, by virtue of the simultaneous evacuation of the air and of the liquids, with consequent reduction of the hospitalization times of the patient.

A further advantage of the thoracic drainage system according to the invention resides in that it facilitates its management, and more generally the management of the thoracic drainage system, and minimizes the intensity of the pain felt by the patient.

Furthermore, the drainage catheter according to the invention can be provided to any drainage system, be it with one or more collection chambers, be it provided or not with valves for the application and adjustment of suction, be it equipped or not with a vacuum unit or connected to the centralized hospital vacuum system. Said coaxial drainage catheter performs its function regardless of the collection system to which it is connected.

The invention thus conceived is susceptible of numerous modifications and variations, all of which are within the scope of the appended claims; by way of nonlimiting example, the person skilled in the art understands without effort that it is also possible to provide a mechanism or a valve for interrupting the flow of fluids in input or for interrupting the flow of liquids and air in output. Furthermore, all the details may be replaced with other technically equivalent elements.

In practice, the materials used, as well as the contingent shapes and dimensions, may be any according to the requirements and the state of the art.

To conclude, the scope of the protection of the claims must not be limited by the illustrations or preferred embodiments shown in the description by way of example, but rather the invention is defined by the appended claims. J Z

The disclosures in Italian Patent Application No. 102016000124793 (UA2016A008934) from which this application claims priority are Z preferred to.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A thoracic drainage system (1), comprising a drainage catheter (10) that is adapted to be inserted in the pleural space of a patient in order to drain air and/or liquids that are present in said pleural space, and a drain tube (50), which is connected to said drainage catheter (10) by means of a connector (30) which is adapted to make said air and/or said drained liquids flow out via said drainage catheter (10) to a collection chamber (60), wherein said drainage catheter (10) comprises, at its distal end (11), a plurality of longitudinally extended channels (13, 14) which are open radially toward the outside of said drainage catheter (10) and are adapted to drain said liquids that are present in said pleural space, and, at its proximal end (12), at least one port (15, 150) for the drainage of said liquids, in which said open channels (13, 14) merge, said drainage catheter (10) comprising a port (16) for the drainage of said air that is present in said pleural space, said port (16) for the drainage of said air comprising, at said distal end (11) of said drainage catheter (10), a plurality of radial holes (17) adapted to be crossed by said air that is present in said pleural space, **characterized in that** said drain tube (50) comprises, substantially along its entire longitudinal extension, a first outflow port (51) which is connected, by means of said connector (30), to said at least one port (15, 150) for the drainage of said liquids and a second outflow port (52) which is connected, by means of said connector (30), to said port (16) for the drainage of said air.

2. The thoracic drainage system (1) according to claim 1, **characterized in that** at least some of said radial holes (17) of said port (16) for the drainage of said air are directed toward at least one of said open channels (13, 14).

3. The thoracic drainage system (1) according to claim 1 or 2, **characterized in that** said drainage catheter (10) has, at said distal end (11), a substantially circular cross-section divided into two circular sectors, a first circular sector accommodating said port (16) for the drainage of said air, a second circular sector accommodating said plurality of open channels (13, 14).

4. The thoracic drainage system (1) according to claim 1 or 2 or 3, **characterized in that** said drainage catheter (10) has, at said proximal end (12), a substantially circular cross-section that is divided into two circular sectors, a first circular sector accommodating said port (16) for the drainage of said air, a second circular sector accommodating said port (15) for the drainage of said liquids into which said open channels (13, 14) merge.

5. The thoracic drainage system (1) according to claim 1 or 2 or 4, **characterized in that** said drainage catheter (10) has, at said distal end (11), a substantially circular cross-section, said port (16) for the drainage of said air being arranged centrally with respect to said substantially circular cross-section, said plurality of open channels (13, 14) being provided so as to be radially external to said port (16) for the drainage of said air.

6. The thoracic drainage system (1) according to one or more of the preceding claims, **characterized in that** said drainage catheter (10) comprises, at said proximal end (12), a pair of ports (150) for the drainage of said liquids which have a substantially semiannular cross-section and are arranged so as to be radially external with respect to said port (16) for the drainage of said air.

7. The thoracic drainage system (1) according to one or more of the preceding claims, **characterized in that** said drainage catheter (10) and/or said drain tube (50) can be obtained by means of processes for the extrusion of materials such as plastic.

8. The thoracic drainage system (1) according to one or more of the preceding claims, **characterized in that** said connector (30) is provided with a first portion (31, 310), which can be associated with said drainage catheter (10) by interference or screw coupling, and a second portion (32), which is opposite with respect to said first portion (31) and can be associated with said drain tube (50) by interference.

9. The thoracic drainage system (1) according to one or more of the preceding claims, **characterized in that** said connector (30) comprises a first connecting duct (31) that is arranged to connect said at least one port (15) for the drainage of said liquids to said first outflow port (51) and a second connecting duct (32) that is arranged to connect said port (16) for the drainage of said air to said second outflow port (52), said second connecting duct (32) comprising, at both of its ends, two cylindrical walls (33, 34) which protrude axially and are arranged to engage respectively in said port (16) for the drainage of said air and in said second outflow port (52).

10. The thoracic drainage system (1) according to one or more of the preceding claims, **characterized in that** said cylindrical walls (33, 34) comprise a guiding bevel (35) for coupling in said port (16) for the drainage of said air and in said second outflow port (52).

## Patentansprüche

1. Thoraxdrainagesystem (1), das einen Drainagekatheter (10) aufweist, der dazu ausgelegt ist, in den Pleuraraum eines Patienten eingeführt zu werden, um Luft und/oder Flüssigkeiten, die in dem Pleuraraum vorhanden sind, zu drainieren, und ein Drainageröhrchen (50), das mittels eines Verbindungsstücks (30) mit dem Drainagekathether (10) verbunden ist, das dazu ausgelegt ist zu veranlassen, dass die Luft und/oder die drainierten Flüssigkeiten aus dem Drainagekatheter (10) zu einer Sammelkammer (60) fließen, wobei der Drainagekatheter (10) an seinem distalen Ende (11) eine Vielzahl länglich erweiterter Kanäle (13, 14) aufweist, die radial zur Außenseite des Drainagekatheters (10) hin offen und dazu ausgelegt sind, die in dem Pleuraraum vorhandenen Flüssigkeiten zu drainieren, und an seinem proximalen Ende (12) wenigstens einen Port (15, 150) zur Drainage der Flüssigkeiten, in den die offenen Kanäle (13, 14) zusammenlaufen, wobei der Drainagekatheter (10) einen Port (16) für die Drainage der Luft aufweist, die in dem Pleuraraum vorhanden ist, wobei der Port (16) für die Drainage der Luft an dem distalen Ende (11) des Drainagekatheters (10) eine Vielzahl radialer Löcher (17) aufweist, die dazu ausgelegt sind, von der Luft, die in dem Pleuraraum vorhanden ist, durchquert zu werden,
**dadurch gekennzeichnet, dass**
das Drainageröhrchen (50) im Wesentlichen entlang seiner gesamten Längsausdehnung einen ersten Ausflussport (51) aufweist, der mittels des Verbindungsstücks (30) mit dem wenigstens einen Port (15, 150) zur Drainage der Flüssigkeiten verbunden ist, und einen zweiten Ausflussport (52), der mittels des Verbindungsstücks (30) mit dem Port (16) zur Drainage der Luft verbunden ist.

2. Thoraxdrainagesystem (1) nach Anspruch 1, **dadurch gekennzeichnet, dass** wenigstens einige der radialen Löcher (17) des Ports (16) zur Drainage der Luft in Richtung wenigstens eines der offenen Kanäle (13, 14) gerichtet sind.

3. Thoraxdrainagesystem (1) nach Anspruch 1 oder 2, **dadurch gekennzeichnet, dass** der Drainagekatheter (10) an seinem distalen Ende (11) einen im Wesentlichen kreisförmigen Querschnitt hat, der in zwei Kreissektoren geteilt ist, einen ersten Kreissektor, der den Port (16) für die Drainage der Luft aufnimmt, einen zweiten Kreissektor, der die Vielzahl offener Kanäle (13, 14) aufnimmt.

4. Thoraxdrainagesystem (1) nach Anspruch 1 oder 2 oder 3, **dadurch gekennzeichnet, dass** der Drainagekatheter (10) an dem proximalen Ende (12) einen im Wesentlichen kreisförmigen Querschnitt hat, der in zwei Kreissektoren geteilt ist, einen ersten Kreissektor, der den Port (16) für die Drainage der Luft aufnimmt, einen zweiten Kreissektor, der den Port (15) für die Drainage der Flüssigkeiten aufnimmt, in den die offenen Kanäle (13, 14) übergehen.

5. Thoraxdrainagesystem (1) nach Anspruch 1 oder 2 oder 4, **dadurch gekennzeichnet, dass** der Drainagekatheter (10) an dem distalen Ende (11) einen im Wesentlichen kreisförmigen Querschnitt hat, wobei der Port (16) für die Drainage der Luft mittig bezüglich des im Wesentlichen kreisförmigen Querschnitts angeordnet ist, wobei die Vielzahl offener Kanäle (13, 14) so vorgesehen sind, dass sie radial außerhalb des Ports (16) für die Drainage der Luft sind.

6. Thoraxdrainagesystem (1) nach einem oder mehreren der vorhergehenden Ansprüche, **dadurch gekennzeichnet, dass** der Drainagekatheter (10) an dem proximalen Ende (12) ein Paar von Ports (150) für die Drainage der Flüssigkeiten aufweist, die einen im Wesentlichen halbringförmigen Querschnitt haben und so angeordnet sind, dass sie bezüglich des Ports (16) für die Drainage der Luft radial außerhalb sind.

7. Thoraxdrainagesystem (1) nach einem oder mehreren der vorhergehenden Ansprüche, **dadurch gekennzeichnet, dass** der Drainagekatheter (10) und/oder das Drainageröhrchen (50) mittels Verfahren zum Extrudieren von Materialien, wie zum Beispiel Kunststoff, erhalten werden können.

8. Thoraxdrainagesystem (1) nach einem oder mehreren der vorhergehenden Ansprüche, **dadurch gekennzeichnet, dass** das Verbindungsstück (30) mit einem ersten Abschnitt (31, 310) versehen ist, der durch Interferenz oder Schraubverbindung mit dem Drainagekatheter (10) verbunden werden kann, und einem zweiten Abschnitt (32), der zu dem ersten Abschnitt (31) entgegengesetzt ist und durch Interferenz mit dem Drainageröhrchen (50) verbunden werden kann.

9. Thoraxdrainagesystem (1) nach einem oder mehreren der vorhergehenden Ansprüche, **dadurch gekennzeichnet, dass** das Verbindungsstück (30) eine erste Verbindungsleitung (31) aufweist, die dazu ausgelegt ist, den wenigstens einen Port (15) für die Drainage der Flüssigkeiten mit dem ersten Ausflussport (51) zu verbinden, und eine zweite Verbindungsleitung (32), die dazu ausgelegt ist, den Port (16) für die Drainage der Luft mit dem zweiten Ausflussport (52) zu verbinden, wobei die zweite Verbindungsleitung (32) an beiden ihrer Enden zwei zylindrische Wände (33, 34) aufweist, die axial vorstehen und dazu ausgelegt sind, in den Port (16) für die Drainage der Luft bzw. in den zweiten Ausflussport (52) einzugreifen.

10. Thoraxdrainagesystem (1) nach einem oder mehreren der vorhergehenden Ansprüche, **dadurch gekennzeichnet, dass** die zylindrischen Wände (33, 34) eine Führungsschräge (35) zum Einkoppeln in den Port (16) für die Drainage der Luft und in den zweiten Ausflussport (52) aufweisen.

## Revendications

1. Système de drainage thoracique (1), comprenant un cathéter de drainage (10) qui est adapté pour être inséré dans l'espace pleural d'un patient afin d'évacuer l'air et/ou les liquides qui sont présents dans ledit espace pleural, et un tube drain (50), qui est connecté audit cathéter de drainage (10) au moyen d'un connecteur (30) qui est adapté pour faire s'écouler ledit air et/ou lesdits liquides évacués, via ledit cathéter de drainage (10), vers une chambre collectrice (60),
dans lequel ledit cathéter de drainage (10) comprend, à son extrémité distale (11), une pluralité de canaux (13, 14) s'étendant longitudinalement, qui sont ouverts radialement vers l'extérieur dudit cathéter de drainage (10) et sont adaptés pour évacuer lesdits liquides qui sont présents dans ledit espace pleural, et, à son extrémité proximale (12), au moins un orifice (15, 150) pour le drainage desdits liquides, dans lequel lesdits canaux ouverts (13, 14) se rejoignent, ledit cathéter de drainage (10) comprenant un orifice (16) pour l'évacuation dudit air qui est présent dans ledit espace pleural, ledit orifice (16) pour l'évacuation dudit air comprenant, à ladite extrémité distale (11) dudit cathéter de drainage (10), une pluralité de trous radiaux (17) adaptés pour être traversés par ledit air qui est présent dans ledit espace pleural, **caractérisé en ce que** ledit tube drain (50) comprend, essentiellement le long de toute son extension longitudinale, un premier orifice de décharge (51) qui est connecté, au moyen dudit connecteur (30), audit au moins un orifice (15, 150) pour l'évacuation desdits liquides et un second orifice de décharge (52) qui est connecté, au moyen dudit connecteur (30), audit orifice (16) pour l'évacuation dudit air.

2. Système de drainage thoracique (1) selon la revendication 1, **caractérisé en ce qu'**au moins certains desdits trous radiaux (17) dudit orifice (16) pour l'évacuation dudit air sont dirigés vers au moins l'un desdits canaux ouverts (13, 14).

3. Système de drainage thoracique (1) selon la revendication 1 ou 2, **caractérisé en ce que** ledit cathéter de drainage (10) présente, à ladite extrémité distale (11), une section transversale essentiellement circulaire divisée en deux secteurs circulaires, un premier secteur circulaire intégrant ledit orifice (16) pour l'évacuation dudit air, un second secteur circulaire intégrant ladite pluralité de canaux ouverts (13, 14).

4. Système de drainage thoracique (1) selon la revendication 1 ou 2 ou 3, **caractérisé en ce que** ledit cathéter de drainage (10) présente, à ladite extrémité proximale (12), une section transversale essentiellement circulaire qui est divisée en deux secteurs circulaires, un premier secteur circulaire intégrant ledit orifice (16) pour l'évacuation dudit air, un second secteur circulaire intégrant ledit orifice (15) pour l'évacuation desdits liquides, dans lequel lesdits canaux ouverts (13, 14) se rejoignent.

5. Système de drainage thoracique (1) selon la revendication 1 ou 2 ou 4, **caractérisé en ce que** ledit cathéter de drainage (10) présente, à ladite extrémité distale (11), une section transversale essentiellement circulaire, ledit orifice (16) pour l'évacuation dudit air étant disposé centralement par rapport à ladite section transversale essentiellement circulaire, ladite pluralité de canaux ouverts (13, 14) étant placés de manière à être externes radialement par rapport audit orifice (16) pour l'évacuation dudit air.

6. Système de drainage thoracique (1) selon une ou plusieurs des revendications précédentes, **caractérisé en ce que** ledit cathéter de drainage (10) comprend, à ladite extrémité proximale (12), une paires d'orifices (150) pour l'évacuation desdits liquides, qui présentent une section transversale essentiellement semi-annulaire et sont disposés de manière à être externes radialement par rapport audit orifice (16) pour l'évacuation dudit air.

7. Système de drainage thoracique (1) selon une ou plusieurs des revendications précédentes, **caractérisé en ce que** ledit cathéter de drainage (10) et/ou ledit tube drain (50) peut/peuvent être obtenu(s) au moyen de processus pour l'extrusion de matériaux tels qu'une matière plastique.

8. Système de drainage thoracique (1) selon une ou plusieurs des revendications précédentes, **caractérisé en ce que** ledit connecteur (30) est doté d'une première partie (31, 310) qui peut être associée audit cathéter de drainage (10) par accouplement par serrage ou vis, et d'une seconde partie (31), qui est opposée par rapport à ladite première partie (31) et peut être associée par ajustement serré audit tube drain (50).

9. Système de drainage thoracique (1) selon une ou plusieurs des revendications précédentes, **caractérisé en ce que** ledit connecteur (30) comprend un premier conduit de connexion (32) qui est disposé pour connecter ledit au moins un orifice (15) pour l'évacuation desdits liquides audit premier orifice de décharge (51) et un second conduit de connexion (32) qui est disposé pour connecter ledit orifice (16) pour l'évacuation dudit air audit second orifice de décharge (52), ledit second conduit de connexion (32) comprenant, à ses deux extrémités, deux parois cylindriques (33, 34) qui font saillie axialement et sont disposées pour s'engager respectivement dans ledit orifice (16) pour l'évacuation dudit air et dans ledit second orifice de décharge (52).

10. Système de drainage thoracique (1) selon une ou plusieurs des revendications précédentes, **caractérisé en ce que** lesdites parois cylindriques (33, 34) comprennent un biseau de guidage (35) pour l'accouplement dans ledit orifice (16) pour l'évacuation dudit air et dans ledit second orifice de décharge (52).
